# EUROPÄISCHE PATENTSCHRIFT

(11) **EP 2 144 597 B1**
(45) Veröffentlichungstag und Bekanntmachung des Hinweises auf die Patenterteilung: **26.01.2011**
(21) Anmeldenummer: 07856589.2
(22) Anmeldetag: 12.12.2007
(51) Int. Cl.: A61K 8/67, A61K 8/37, A61Q 19/08, A61Q 19/10, A61K 31/07, A61K 31/203

(54) **WIRKSTOFF-STABILISIERENDE O/W-EMULSIONEN II**
OIL-IN-WATER EMULSIONS II WHICH STABILIZE THEIR ACTIVE INGREDIENT
ÉMULSIONS HUILE DANS L'EAU II STABILISANT LEUR PRINCIPE ACTIF

(30) Priorität: 28.02.2007 DE 102007010111; 10.12.2007 DE 102007059703
(43) Veröffentlichungstag der Anmeldung: 20.01.2010
(73) Patentinhaber: Henkel AG & Co. KGaA, 40589 Düsseldorf (DE)
(72) Erfinder: SCHELGES, Heike, 47877 Willich (DE); PLÖTZNER, Renate, 42781 Haan (DE)
(86) Internationale Anmeldenummer: PCT/EP2007/010841
(87) Internationale Veröffentlichungsnummer: WO 2008/104215

(56) Entgegenhaltungen:
- EP-A- 0 163 806
- EP-A1- 0 985 404
- WO-A-2005/097055
- DE-A1-102004 031 939
- DE-A1-102005 063 179
- US-A1- 2004 167 046
- US-A1- 2005 255 130

## Beschreibung

Die vorliegende Anmeldung betrifft Öl-in-Wasser-Formulierungen (O/W-Formulierungen), die mindestens ein Retinoid enthalten, deren Verwendung als Hautpflegemittel, insbesondere als Antifalten-Mittel und/oder Anti-Akne-Mittel und/oder als Mittel gegen unreine Haut, und ein Verfahren zu deren Stabilisierung.

### Stand der Technik

O/W-Formulierungen, die Retinoid als Wirkstoff enthalten, sind dem Fachmann seit langem bekannt. WO 2005/097055 A2 und DE 102004031939 A1 offenbaren Öl-in-Wasser-Zusammensetzungen, enthaltend eine Wachskomponente mit einem Schmelzpunkt von wenigstens 30 °C, ausgewählt aus der Gruppe der Pentaerythritester, der Dipentaerythritester und/oder der Tripentaerythritester, eine bei 25°C flüssige Ölkomponente und Wasser. Retinoide sind als optionaler Bestandteil offenbart. US 2004/0167046 A1 offenbart eine Öl-in-Wasser-Emulsion mit Pentaerythrityltetraoctanoat, einem bei Raumtemperatur flüssigen Öl mit einem Schmelzpunkt unter 30 °C. Retinoide sind als optionaler Bestandteil offenbart. EP 163806 A1 offenbart kosmetische Zusammensetzungen, die als Moisturizer, Emollient und Emulsionsstabilisator bei Raumtemperatur flüssige Oligomere von teilweise verestertem Pentaerythritol enthalten.

Ein großes Problem, mit dem der Fachmann bei der Formulierung von Retinoid-haltigen Kosmetika konfrontiert ist, besteht in der Instabilität der Retinoide, beispielsweise gegenüber Oxidation. US 2005/0255130 A1 offenbart die Stabilisierung von Retinoiden in wasserhaltigen Emulsionen, indem das Retinoid in einer Mischung aus wasserfreiem Ethanol und einem Ester, vorzugsweise Benzoesäureester, Isopropylmyristat, Isopropylpalmitat oder Diisopropyladipat, als Co-Lösemittel solubilisiert wird.

EP 0 985 404 beschreibt O/W-Emulsionen enthaltend Vitamin-A-Fellsäure-Ester, welche gegen Oxidation und Hydrolyse geschülzt sind durch nichdionische hydrophile Emulgatoren und nichtionische amphiphile Polymere.

Aufgabe der vorliegenden Erfindung war es, Retinoid-haltige O/W-Emulsionen mit zur Verfügung zu stellen, die langzeitstabil sind.

Eine weitere Aufgabe war es, O/W-Emulsionen mit mindestens einem Retinoid zu formulieren, die ein angenehmes, sensorisch leichtes Profil und gute Pflegeeffekte aufweisen und bei Applikation auf der Haut nicht brechen.

Ein weiterer Aspekt der Aufgabe war es, möglichst irritationsfreie O/W-Emulsionen mit mindestens einem Retinoid bereitzustellen.

Überraschend wurde nun gefunden, dass sich diese Eigenschaften erreichen lassen, wenn man O/W-Emulsionen formuliert, die eine Kombination von ausgewählten Wachsen und Ölen enthalten.

### Beschreibung

Gegenstand der vorliegenden Anmeldung sind Öl-in-Wasser-Emulsionen, enthaltend (a) mindestens Retinoid, (b) 0,1 - 10 Gew.-% wenigstens einer Wachskomponente mit einem Schmelzpunkt von wenigstens 30°C, ausgewählt aus der Gruppe der Pentaerythritester, der Dipentaerythritester und/oder der Tripentaerythritester sowie Mischungen hiervon, (c) 1 - 30 Gew.-% wenigstens einer bei 25°C flüssigen Ölkomponente und (d) 40 - 95 Gew.-% Wasser. Zusammensetzungen dieser Art sind langzeitstabil und hinterlassen bei der Anwendung ein glattes und weiches Gefühl mit sehr guten Pflegeeigenschaften. Sie sind leicht verteilbar, ziehen gut auf die Haut auf, hinterlassen ein geringes öliges oder fettendes, sondern vielmehr samtiges Hautgefühl.

In einer bevorzugten Ausführungsform enthalten die erfindungsgemäßen Zusammensetzungen keine zusätzlichen anionischen und kationischen Tenside/Emulgatoren. Dadurch haben die Zusammensetzungen ein geringes Irritationspotenzial.

Die erfindungsgemäßen O/W-Emulsionen weisen vorzugsweise eine Viskosität von 20000 bis 500000 mPa s, bevorzugt 50000 bis 80000 mPa s, auf, gemessen bei 20°C mit einem Brookfield-Viskosimeter RVF, Spindel TE, mit Helipath bei 4 Umdrehungen pro Minute.

Pentaerythritester, Dipentaerythritester und/oder Tripentaerythritester als Wachskomponente Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30 °C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß obligatorisch ist eine Wachskomponente oder ein Gemisch von Wachskomponenten, die bei 30 °C oder darüber schmilzt/schmelzen und ausgewählt ist/sind aus der Gruppe der Ester des Pentaerythrits, des Dipentaerythrits und des Tripentaerythrits. Die Ester des Pentaerythrits sind bevorzugt.

Die vorgenannten Ester des Pentaerythrits, Dipentaerythrits und/oder Tripentaerythrits sind in den erfindungsgemäßen Zusammensetzungen in einer Gesamtmenge von 0,1 - 10 Gew.-% enthalten. In einer bevorzugten Ausführungsform der Erfindung liegt der Gehalt dieser Pentaerythrit-basierten Wachskomponente bei 0,2 - 5 Gew.-%, besonders bevorzugt bei 0,5 - 4 Gew.-% und insbesondere 1 - 2 Gew.-%, jeweils bezogen auf die Gesamtzusammensetzung. In den genannten Mengenbereichen ist das sensorische Gesamtprofil der erfindungsgemäßen Öl-in-Wasser-Emulsionen optimal.

Eine weitere bevorzugte Ausführungsform der erfindungsgemäßen Öl-in-Wasser-Emulsionen ist dadurch gekennzeichnet, dass die Wachskomponente oder das Gemisch aus Wachskomponenten (b) einen Schmelzpunkt zwischen 40°C und 80 °C aufweist, vorzugsweise zwischen 40 und 60 °C, da sich in diesem Bereich die besten sensorischen Effekte ergeben.

In einer weiteren bevorzugten Ausführungsform der Erfindung ist die Wachskomponente (b) ausgewählt aus der Gruppe der Ester gesättigter oder ungesättigter und/oder verzweigter oder unverzweigter C6-C24-Fettsäuren, vorzugsweise C14-C22-Fettsäuren des Pentaerythrits, des Dipentaerythrits und/ oder Tripentaerythrits, wobei weniger als 0.3 Gew.-% C17-Fettsäureester enthalten sind. Die Ester müssen einen Schmelzpunkt von wenigstens 30°C aufweisen. Es kann sich auch um Mischester, beispielsweise langkettiger und kurzkettiger Fettsäuren, handeln, solange sie den geforderten Schmelzpunkt aufweisen. Eine weitere bevorzugte Ausführungsform der O/W-Emulsion ist dadurch gekennzeichnet, dass die Wachskomponente (b) ausgewählt ist aus der Gruppe der Ester, die durch Umsetzung des Pentaerythrits oder Dipentaerythrits mit einem Fettsäuregemisch, enthaltend 40 - 50 Gew.-% C16-Fettsäure und 45 - 55 Gew.-% C18-Fettsäure, erhältlich sind. Besonders bevorzugt sind Ester linearer, unverzweigter C16/C18-Fettsäuren.

Eine weitere bevorzugte Ausführungsform der erfindungsgemäßen Öl-in-Wasser-Emulsionen enthält wenigstens eine Wachskomponente (b), ausgewählt aus der Gruppe der Ester des Pentaerythrits mit einem Anteil an 5 - 35 Gew.-% Monoester, 20 - 50 Gew.-% Diester und 25 - 50 Gew.-% Triester, und ggf. Tetraester. Ganz besonders bevorzugt sind Ester, die durch Umsetzung des Pentaerythrits mit einem Fettsäuregemisch, enthaltend 40 - 50 Gew.-% C16-Fettsäure und 45 - 55 Gew.-% C18-Fettsäure, erhältlich sind und die folgende Esterverteilung aufweisen: 12 - 19 Gew.-% Monoester, 25 - 35 Gew.-% Diester, 30 - 40 Gew.-% Triester und 6 - 11 Gew.-% Tetraester.

Erfindungsgemäß besonders bevorzugt ist das Handelsprodukt Cutina PES (ex Cognis), INCI-Bezeichnung Pentaerythrityl Distearate

### Ölkörper

Die erfindungsgemäßen Öl-in-Wasser-Emulsionen enthalten weiterhin obligatorisch 1 - 30 Gew.-%, bezogen auf die Gesamtzusammensetzung, mindestens einer bei 25°C flüssigen Ölkomponente. Vorzugsweise ist/sind die Ölkomponente(n) in einer Gesamtmenge von 3 - 20 Gew.-%, insbesondere 5 - 15 Gew.-% und besonders bevorzugt 7 - 12 Gew.-%, bezogen auf das Gesamtgewicht der erfindungsgemäßen Öl-in-Wasser-Emulsion, enthalten.

Als Ölkörper sind beispielsweise die nachstehend genannten Verbindungsklassen geeignet, soweit diese bei 25°C flüssig sind. Hierzu zählen u.a. Guerbetalkohole auf Basis von Fettalkoholen mit 6 - 18, vorzugsweise 8 - 10 Kohlenstoffatomen, Ester von linearen oder verzweigten, gesättigten oder ungesättigten C6-C22-Fettsäuren mit linearen oder verzweigten, gesättigten oder ungesättigten C6-C22-Fettalkoholen, insbesondere 2-Ethylhexanol. Beispielhaft seien genannt Hexyllaurat, Myristylisostearat, Myristyloleat, Cetylisostearat, Cetyloleat, Stearylisostearat, Stearyloleat, Isostearylisononanoat, Isostearylmyristat, Isostearylpalmitat, Isostearylstearat, Isostearylisostearat, Isostearyloleat, Oleylmyristat, Oleylisostearat, Oleyloleat, Oleylerucat, Erucylisostearat, Erucyloleat, Cococaprylat/caprat. Weitere geeignete Ester sind z.B. Ester von C18-C38-Alkylhydroxycarbonsäuren mit linearen oder verzweigten, gesättigten oder ungesättigten C6-C22-Fettalkoholen, Ester von linearen und/oder verzweigten, gesättigten oder ungesättigten Fettsäuren mit mehrwertigen Alkoholen (wie z. B. Propylenglycol, Dimerdiol oder Trimertriol) und/oder Guerbetalkoholen, weiterhin Triglyceride und Triglyceridmischungen, Mono-/ Di-/Triglyceridmischungen, Ester von C6-C22-Fettalkoholen und/oder Guerbetalkoholen mit aromatischen Carbonsäuren, insbesondere Benzoesäure, Ester von C2-C12-Dicarbonsäuren mit linearen oder verzweigten, gesättigten oder ungesättigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare Dialkylcarbonate, Guerbetcarbonate auf Basis von Fettalkoholen mit 6 bis 18, vorzugsweise 8 bis 10 C-Atomen, Ester der Benzoesäure mit linearen und/oder verzweigten C6-C22-Alkoholen (z. B. Cetiol AB), lineare oder verzweigte symmetrische oder unsymmetrische Dialkylether mit 6 bis 22 Kohlenstoffatomen pro Alkylgruppe, wie z. B. Di-n-octyl-ether (Cetiol OE), Ringöffnungsprodukte von epoxidierten Fettsäureestern mit Polyolen, Kohlenwasserstoffe wie Paraffin- oder Mineralöle, Siliconöle, Oligo-oder Polyalphaolefine, Dialkylcarbonate, Ester aus C8-C24-Fettsäuren und C8-C24-Fettalkoholen, sowie Mischungen dieser Substanzen.

Eine erfindungsgemäß bevorzugte Gruppe von Ölen sind die Dialkylcarbonate. Die Dialkylcarbonate können symmetrisch oder unsymmetrisch, verzweigt oder unverzweigt, gesättigt oder ungesättigt sein und lassen sich nach Umesterungsreaktionen, die aus dem Stand der Technik hinlänglich bekannt sind, herstellen. Erfindungsgemäß besonders geeignet sind Dialkylcarbonate mit Alkylketten, die 6 bis 24 Kohlenstoffatome aufweisen, insbesondere Di-n-octylcarbonat oder Di-(2-ethylhexyl)carbonat oder ein Gemisch dieser Substanzen. Unter diesen ist das Di-n-Octylcarbonat. bevorzugt.

Weitere bevorzugte Öle sind ausgewählt aus den Estern von linearen oder verzweigten, gesättigten oder ungesättigten C6-C22-Fettsäuren mit linearen oder verzweigten, gesättigten oder ungesättigten C6-C22-Fettalkoholen, insbesondere Hexyllaurat, Isostearylisononanoat, Isostearylpalmitat, lsostearylstearat, Isostearylisostearat und Cococaprylat/caprat.

Weitere bevorzugte Öle sind ausgewählt aus den Triglyceriden, das heißt, den Glyceryltriestern, und Triglyceridmischungen, die bei 25 °C flüssig sind. Hierzu zählen besonders bevorzugt die Glyceryltriester der Octansäure (Caprylic Triglyceride) und der Decansäure (Capric Triglyceride). Weitere bevorzugte Öle sind ausgewählt aus pflanzlichen Ölen, insbesondere Distelöl, Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls. Ein aufgrund seiner relativ starken Polarität besonders bevorzugtes pflanzliches Öl ist Distelöl.

Weitere bevorzugte Öle sind ausgewählt aus den Estern der Benzoesäure mit linearen und/oder verzweigten C6-C22-Alkoholen, insbesondere aus Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD.

Weitere bevorzugte Öle sind ausgewählt aus den verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16, Guerbitol^{®} T 16), Octyldodecanol (Eutanol^{®} G, Guerbitol^{®} 20), 2-Ethylhexylalkohol und die Handelsprodukte Guerbitol^{®} 18, Isofol^{®} 12, Isofol^{®} 16, Isofol^{®} 24, Isofol^{®} 36, Isocarb^{®} 12, Isocarb^{®} 16 oder Isocarb^{®} 24, wobei Hexyldecanol besonders bevorzugt ist.

Mit Ausnahme der Kohlenwasserstoffe, wie Paraffin- oder Mineralöle, Oligo- oder Polyalphaolefine, und der Siliconöle werden die übrigen vorstehend aufgeführten Substanzklassen eher zu den polaren Ölen gerechnet. Kohlenwasserstoffe, wie Paraffin- oder Mineralöle, Oligo- oder Polyalphaolefine, und die Siliconöle zählen dagegen eher zu den unpolaren Ölen. Überraschend wurde festgestellt. dass sich besonders lager- und temperaturstabile Öl-in-Wasser-Emulsionen formulieren lassen, wenn die Ölkomponente mindestens ein polares Öl und mindestens ein unpolares Öl enthält, wobei die polaren Öle gegenüber den unpolaren Ölen im Überschuss vorliegen. Bevorzugt beträgt dabei das Gewichtsverhältnis der Gesamtheit der polaren Öle zur Gesamtheit der unpolaren Öle 5:1 bis 20:1, bevorzugt 6:1 - 10:1 und besonders bevorzugt 7:1 - 8:1.

Erfindungsgemäß besonders bevorzugte polare Öle sind ausgewählt aus Di-n-Octylcarbonat, Cococaprylat/caprat, Hexyllaurat, Isostearylisononanoat, Isostearylpalmitat, Isostearylstearat, Isostearylisostearat, Caprylic Triglyceride, Capric Triglyceride, Distelöl, Benzoesäure-C12-C15-alkylester, Hexyldecanol, Octyldodecanol, sowie Mischungen dieser Öle.

Eine besonders bevorzugte Ölmischung umfasst Di-n-Octylcarbonat und Cococaprylat/caprat. Überraschend wurde festgestellt, dass diese Ölmischung die Stabilität des Retinoids vorteilhaft unterstützt und darüber hinaus nicht-komedogen wirkt, was insbesondere vorteilhaft für das bevorzugte Anwendungsgebiet der erfindungsgemäßen Zusammensetzungen ist, nämlich die Behandlung unreiner Haut oder Aknehaut. Als nicht-komedogene Ölkörper gelten ansonsten eher die Siliconöle, die aber nicht zu den polaren Ölen gerechnet werden.

Diese polaren Öle und Ölmischungen werden erfindungsgemäß besonders bevorzugt mit Polydimethylsiloxanen (INCl): Dimethicone) als unpolarer Ölkomponente kombiniert.

Weitere erfindungsgemäß als eher unpolare Öle einsetzbare Kohlenwasserstoffe weisen eine Kettenlänge von vorzugsweise 8 bis 40 C-Atomen aus. Sie können verzweigt oder unverzweigt sein, gesättigt oder ungesättigt. Unter diesen sind verzweigte, gesättigte C8-C40-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzungen, die Alkane mit 10 bis 30, vorzugsweise 12 bis 20, und besonders bevorzugt 16 bis 20 Kohlenstoffatomen aufweisen, sind besonders geeignet, und unter diesen ist ein Gemisch aus Alkanen, das wenigstens 10 Gew.-% verzweigte Alkane, bezogen auf die Gesamtmenge der Alkane, enthält, besonders bevorzugt. Bevorzugt handelt es sich um verzweigte, gesättigte Alkane. Besonders gut geeignet sind Gemische aus Alkanen, die mehr als 1 Gew.-% 5,8-Diethyldodecan und/oder mehr als 1 Gew.-% Didecen enthalten.

### Retinoide

Zu den Retinoiden werden im Sinne der vorliegenden Erfindung Retinol (Vitamin A₁), die C₂-C₂₂-Fettsäureester des Retinols (= Retinylester, insbesondere Retinylpalmitat und Retinylacetat), 3,4-Didehydroretinol (Vitamin A₂), Retinal und seine Isomere, *all-trans*-Retinsäure, 9-*cis*-Retinsäure und 13-*cis*-Retinsäure (Tretinoin), die Ester der Retinsäure sowie weitere verwandte Substanzen (synthetische Retinoide) gezählt, die für ihre vielfältige biologische Wirkung insbesondere auf Wachstum und Differenzierung bekannt sind, sowie weiterhin Substanzen, die eine spezifische biologische Wirkung durch die Bindung an die Retinoid-Rezeptoren RAR (retinoid acid receptor) und RXR (retinoid X receptor) aufweisen und deren Aktivierung verursachen. Synthetische Retinoide werden in drei Gruppen eingeteilt: nichtaromatisch (z. B. *lsotretinoin*)*,* monoaromatisch (z.B. Acitretin) und polyaromatisch (so genannte Arotinoide, z. B. Tazarotene, die eine spezifische Wirkung auf einzelne Retinoid-Rezeptoren haben). Erfindungsgemäß besonders bevorzugte Retinoide sind Retinol und die C₂-C₂₂-Fettsäureester des Retinols, insbesondere Retinylpalmitat und Retinylacetat.

Retinoide werden bevorzugt als Antifaltenwirkstoff und/oder als Anti-Akne-Wirkstoff und/oder als Wirkstoff gegen unreine Haut verwendet.

Erfindungsgemäß bevorzugte Öl-in-Wasser-Emulsionen enthalten mindestens ein Retinoid in einer Gesamtmenge von 0,01 - 2 Gew.-%, bevorzugt 0,02 - 1 Gew.-%, besonders bevorzugt 0,05 - 0,5 Gew.-%, außerordentlich bevorzugt 0,07 - 0,1 Gew.-%, jeweils bezogen auf die gesamte Öl-in-Wasser-Emulsion.

### Gelbildner

Erfindungsgemäß bevorzugte Öl-in-Wasser-Emulsionen enthalten mindestens einen Gelbildner, bevorzugt einen Hydrogelbildner, der besonders bevorzugt aus der Gruppe der Homopolymere oder Copolymere von Acrylsäure und/oder Acrylamid und/oder Acryloyldimethyltaurat und deren Salzen oder aus einem beliebigen Gemisch dieser Substanzen ausgewählt ist. Häufig sind die marktüblichen Hydrogelbildner als wasserbasierte Polymerzubereitungen konfektioniert, die gegebenenfalls ein nichtionisches Tensid/Emulgator und gegebenenfalls ein Öl enthalten. Zu den erfindungsgemäß bevorzugten Gelbildnern gehören im Handel erhältliche Substanzen oder Polymerzubereitungen, wie beispielsweise Sepigel 305, lNCl: Polyacrylamid (und) C13-14 Isoparaffin (und) Laureth-7; Sepigel 501, lNCl: Acrylamid Copolymer (und) Mineralöl (und) C13-14 Isoparaffin (und) Polysorbate 85. Sepigel 502, INCl: C13-14 Isoparaffin (und) Isostearyl lsostearat (und) Sodium Polyacrylate (und) Polyacrylamid (und) Polysorbate 60; Simulgel 600, lNCl: Acrylamid/Sodium Acryloyldimethyltaurate Copolymer (und) Isohexadecan (und) Polysorbat 80; Simulgel 800, INCl: Sodium Polyacryloyldimethyl Taurate (und) Isohexadecan (und) Sorbitan Oleat; Simulgel EG, lNCl: Sodium Acrylate/Acryloyldimethyl Taurate Copolymer (und) Isohexadecan (und) Polysorbate 80; Simulgel EG-SL (oder auch Simulgel EPG), lNCl: Sodium Acrylate/Acryloyldimethyl Taurate Copolymer (und) Polyisobutene (und) Caprylyl/Capryl Glucosid; Simulgel NS, lNCl: Hydroxyethyl Acrylate (und) Sodium Acryloyldimethyl Taurate Copolymer (und) Squalane (und) Polysorbate 60; Aristoflex AVC, lNCl: Ammonium Acryloyldimethyltaurate/VP Copolymer; Aristoflex AVC-1, lNCl: Ammonium Acryloyldimethyltaurat / Vinyl Formamid Copolymer; Aristoflex HMB, lNCl: Ammonium Acryloyldimethyltaurate / Beheneth-25 Methacrylate Copolymer, Salcare SC 91, lNCl: Sodium Acrylate Copolymer (und) Mineralöl (und) PPG-1 Trideceth-6; Salcare AST, INCl: Sodium Acrylate Copolymer (und) Glycine Soja (und) PPG-1 Trideceth-6; Pemulen TR-1, lNCl: Acrylates / C10-30 Alkyl Acrylate Crosspolymer; Pemulen TR-2: Acrylates / C10-30 Alkyl Acrylate Crosspolymer; Carbopol 980, lNCl: Carbomer (z.B. Homopolymere von Acrylsäure, vernetzt mit einem Allylether von Pentaerythritol, einem Allylether von Sucrose oder einem Allylether von Propylen); Carbopol ETD 2020, lNCl: Acrylates / C10-30 Alkyl Acrylate Crosspolymer; Carbopol Ultrez 10, INCl: Carbomer; Rheocare ATH, lNCl: Sodium Polyacrylate (und) Ethylhexyl Stearate (und) Trideceth-6; Rheocare ATC, lNCl: Acrylamid / Sodium Acrylate Copolymer (und) Mineralöl (und) Trideceth-6.

Die Polymere können vernetzt oder unvernetzt sein. Vorzugsweise werden vernetzte Polymere eingesetzt.

Erfindungsgemäß bevorzugt sind Polyacrylate und Polyacryloyldimethyltaurate. Besonders bevorzugt sind Polyacrylate, insbesondere deren Natriumsalze, sowie Polyacryloyldimethyltaurate und deren Natrium- und/oder Ammoniumsalze. Die Natriumsalzform ist dabei besonders bevorzugt. Ein erfindungsgemäß besonders bevorzugtes Polymer ist unter dem Namen Cosmedia SP und Cosmedia SPL (Cognis) im Handel. Weiterhin besonders bevorzugt sind die Polyacryloyldimethyltaurat-Zubereitungen Sepigel 305, Simulgel NS, Simulgel EG und Simulgel EPG (siehe vorstehende Erläuterung). Die Polymere werden erfindungsgemäß bevorzugt in einer Gesamtmenge von 0,05 - 1 Gew.-%, besonders bevorzugt 0,1 - 0,8 Gew.-%, insbesondere von 0,2 - 0,6 Gew.-% und ganz besonders bevorzugt von 0,3 - 0,4 Gew.-%, jeweils bezogen auf die Polymeraktivsubstanz in der Gesamtzusammensetzung, eingesetzt.

Eine bevorzugte Ausführungsform der erfindungsgemäßen Öl-in-Wasser-Emulsion enthält (a) mindestens ein Retinoid, (b) 0,1 - 10 Gew.-% wenigstens eines C16-C18-Esters des Pentaerythrits und/oder des Dipentaerythrits, (c) 1 - 30 Gew.-% wenigstens einer bei 25 °C flüssigen Ölkomponente, (d) 0,05 - 5 Gew.-% wenigstens eines Polyacrylats und (e) 60 - 95 Gew.-% Wasser.

Eine weitere bevorzugte Ausführungsform der erfindungsgemäßen O/W-Emulsion enthält (a) mindestens ein Retinoid, (b) 0,1 - 10 Gew.-% wenigstens eines C16-C18-Esters des Pentaerythrits und/oder des Dipentaerythrits, (c) 1 - 30 Gew.-% wenigstens einer bei 25 °C flüssigen Ölkomponente, (d) 0,05 - 5 Gew.-% wenigstens eines Polyacrylats und (e) 40 - 95 Gew.% Wasser.

Das Polyacrylat (d) ist vorzugsweise ein Natriumpolyacrylat.

Die Ölkomponenten sind auch hier bevorzugt ausgewählt aus den vorstehend beschriebenen Mischungen polarer Öle, darunter besonders bevorzugt Fettsäureester und Dialkylcarbonate, die besonders bevorzugt mit einem Unterschuss unpolarer Öle, wie vorstehend beschrieben, als Gemisch vorliegen.

Eine weitere bevorzugte Ausführungsform der erfindungsgemäßen Öl-in-Wasser-Emulsion enthält (a) mindestens ein Retinoid, (b) 0,1 - 10 Gew.% wenigstens eines C16-C18-Esters des Pentaerythrits und/oder des Dipentaerythrits, (c) 1 - 30 Gew.% wenigstens einer bei 25 °C flüssigen Ölkomponente, (d) 0,05 - 5 Gew-% wenigstens eines Acryloyldimethyltaurats und (e) 40 - 95 Gew.-% Wasser.

Die Ölkomponenten sind auch hier bevorzugt ausgewählt aus den vorstehend beschriebenen Mischungen polarer Öle, darunter besonders bevorzugt Fettsäureester und Dialkylcarbonate, die besonders bevorzugt mit einem Unterschuss unpolarer Öle, wie vorstehend beschrieben, als Gemisch vorliegen.

Eine weitere bevorzugte Ausführungsform der erfindungsgemäßen Öl-in-Wasser-Emulsion enthält (a) mindestens ein Retinoid, (b) 0,5 - 2 Gew.-% wenigstens eines Esters, den man durch Umsetzung des Pentaerythrits und/oder des Dipentaerythrits mit einem Fettsäuregemisch, enthaltend 40 - 50 Gew.-% C16-Fettsäure und 45 - 55 Gew.-% C18-Fettsäure, erhält, (c) 7 - 12 Gew.-% wenigstens einer Ölkomponente, ausgewählt aus bei 25 °C flüssigen Fettsäureestern, Triglyceriden, Dialkylcarbonaten, Kohlenwasserstoffen, Dialkylethern oder einem beliebigen Gemisch dieser Substanzen, (d) 0,5 - 2 Gew-% wenigstens eines Natriumpolyacrylats und (e) 60 - 95 Gew.-% Wasser.

Vorzugsweise sind die unter (b) genannten Ester Pentaerythritester mit folgender Esterverteilung: 12 -19 Gew.-% Monoester, 25 - 35 Gew.-% Diester, 30 - 40 Gew.-% Triester und 6 - 11 Gew-% Tetraester.

Weiterhin wurde überraschend festgestellt, dass die erfindungsgemäßen Öl-in-Wasser-Emulsionen auch die problemlose Einarbeitung von organischen und/oder anorganischen UV-Filtern ermöglichen, unter gleichzeitiger Lager- und Temperaturstabilität, und dabei ein sehr gutes sensorisches Hautgefühl aufweisen. Antifaltenzusammensetzungen werden von vielen Verbrauchern eingesetzt, um die Folgen der Hautalterung durch schädigende UV-Strahlen zu behandeln. Hier ist es besonders sinnvoll, dem Wunsch des Verbrauchers nach größtmöglichem Lichtschutz nachzukommen und die Antifaltenwirkung mit einem Lichtschutzfaktor zu kombinieren. Erfindungsgemäß besonders bevorzugte Öl-in-Wasser-Emulsionen sind deshalb dadurch gekennzeichnet, dass sie mindestens eine anorganische und/oder mindestens eine organische UV-Filtersubstanz enthalten.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt. Die erfindungsgemäß bevorzugten organischen UV-Filter sind ausgewählt aus den Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Die Benzoxazol-Derivate liegen vorteilhaft in gelöster Form in den erfindungsgemäßen kosmetischen Zubereitungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen. Erfindungsgemäß besonders bevorzugte öllösliche UV-Filter sind 1-(4-tert.-Butylphenyl)-3-(4-methoxyphenyl)propan-1,3-dion (Parsol^{®} 1789), 1-Phenyl-3-(4`-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4`-methylbenzophenon, 2,2'-Dihydroxy-4-methoxybenzophenon, 2-(4'-Diethylamino-2'-hydroxybenzoyl)-benzoesäurehexylester (auch: Aminobenzophenon, unter der Bezeichnung Uvinul A Plus bei der Firma BASF erhältlich), 4-Methoxybenzmalonsäuredi-2-ethylhexylester, an Polymere gebundene UV-Filter, z. B. das 3-(4-(2,2-Bis-Ethoxycarbonylvinyl)-phenoxy)propenyl)-methoxysiloxan/Dimethylsiloxan-Copolymer mit der lNCl-Bezeichnung Dimethicodiethylbenzal malonate (CAS-Nr. 207574-74-1, Parsolo^{®} SLX), Triazinderivate, wie z. B. 2,4-Bis-{[4-(2-Ethyl-hexyloxy)-2-hydroxy]-phenyl}-6-(4-methoxyphenyl)-1,3,5-triazin (lNCl: Bis-Ethylhexyloxyphenol Methoxyphenyl Triazin, unter dem Namen Tinosorb S bei CIBA erhältlich), Dioctylbutylamidotriazon (lNCl: Diethylhexyl Butamido Triazone, unter dem Namen Uvasorb^{®} HEB bei Sigma 3V erhältlich), 2,4,6-Trianilino-(p-carbo-2'-ethyl-1'-hexyloxy)-1,3,5-triazin (Ethylhexyl Triazone, Uvinul^{®} T 150), 2-[4,6-Bis(2,4-dimethylphenyl)-1,3,5-triazin-2-yl]-5-(octyloxy)phenol (CAS Nr.: 2725-22-6), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma), die Benzotriazolderivate 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCl-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (lNCl : Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethylhexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten.

Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der lNCl-Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der lNCl-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der lNCl-Bezeichnung Terephthalydene Dicampher Sulfonic Acid (CAS.-Nr.: 90457-82-2, als Mexoryl SX von der Firma Chimex erhältlich).

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'-methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'-methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

Erfindungsgemäß ist mindestens eine organische UV-Filtersubstanz bevorzugt in einer Gesamtmenge von 0,1 - 30 Gew.-%, besonders bevorzugt 0,5 - 20 Gew.-%, außerordentlich bevorzugt 1,0 - 15 Gew.-% und weiter bevorzugt 3,0 - 10 Gew.-%, jeweils bezogen auf die gesamte Zusammensetzung, enthalten.

Erfindungsgemäß ist mindestens eine anorganische UV-Filtersubstanz bevorzugt in einer Gesamtmenge von 0.1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Weiterhin wurde überraschend festgestellt, dass die erfindungsgemäßen Öl-in-Wasser-Emulsionen bevorzugt durch nichtionische Emulgatoren oder Tenside weiter lager-, oxidations- und temperaturstabil gemacht werden können und dabei ein sehr gutes sensorisches Hautgefühl aufweisen. Erfindungsgemäß besonders bevorzugte Öl-in-Wasser-Emulsionen sind deshalb dadurch gekennzeichnet, dass sie mindestens einen nichtionischen Öl-in-Wasser-Emulgator enthalten.

Typische Beispiele für nichtionische Öl-in-Wasser-Emulgatoren sind Fettalkoholpolyglycolether, Polyglycerinester, Fettsäurepolyglycolester, Fettsäureamid-polyglycolether, Fettaminpolyglycolether, alkoxylierte Triglyceride, Mischether, Alk(en)yloligoglykoside bzw. Glucoronsäurederivate - gegebenenfalls partiell oxidiert -, Fettsäure-N-alkylglucamide, Polyolfettsäureester, Zuckerester, Sorbitanester, Polysorbate und Aminoxide. Sofern die nichtionischen Öl-in-Wasser-Emulgatoren Polyglycoletherketten enthalten, können diese eine konventionelle, vorzugsweise jedoch eine eingeengte Homologenverteilung aufweisen.

Bevorzugte nichtionische Öl-in-Wasser-Emulgatoren sind ausgewählt aus ethoxylierten C₈-C₂₄-Alkanolen mit durchschnittlich 5- 100 Mol Ethylenoxid pro Mol, ethoxylierten C₈-C₂₄-Carbonsäuren mit durchschnittlich 5 - 100 Mol Ethylenoxid pro Mol, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern sie einen HLB-Wert von mehr als 7 aufweisen, sowie Mischungen der vorgenannten Substanzen, wobei der gewichtsmittlere HLB-Wert des Öl-in-Wasser-Emulgatorsystems bevorzugt 11 - 17, besonders bevorzugt 12 - 15 und außerordentlich bevorzugt 13 - 14 beträgt. Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 5 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 5 - 100 Mol, vorzugsweise 10 - 30 Mol, Ethylenoxid an technische Fettalkohole mit 12 - 18 Kohlenstoffatomen, wie beispielsweise Kokos-, Palm-, Palmkern- oder Talgfettalkohol, sind geeignet.

Besonders bevorzugte nichtionische Öl-in-Wasser-Emulgatoren sind ausgewählt aus der Gruppe, bestehend aus Ceteth-12, Ceteth-20, Ceteth-30, Steareth-12, Steareth-20, Steareth-30, Laureth-12 und Beheneth-20, sowie Mischungen hiervon.

Die ethoxylierten C₈-C₂₄-Carbonsäuren haben die Formel R¹(OCH₂CH₂)ₙOH, wobei R¹ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 5 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 5 - 100 Mol, vorzugsweise 10 - 30 Mol, Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen. Weiterhin werden vorzugsweise C₈ - C₂₂-Alkylmono- und -oligoglycoside eingesetzt. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Ptantacare^{®} erhältlich sind, enthalten eine glucosidisch gebundene C₈-C₁₆-Alkylgruppe an einem Oligoglucosidrest, dessen mittlerer Oligomerisationsgrad bei 1 - 2 liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Die nichtionischen Öl-in-Wasser-Emulgatoren können u.a. auch durch handelsübliche gelbildende Polymerzubereitungen eingetragen werden, wenn auch nur in geringen Mengen, bezogen auf die erfindungsgemäße Öl-in-Wasser-Emulsion.

Besonders bevorzugte erfindungsgemäße Öl-in-Wasser-Emulsionen sind dadurch gekennzeichnet, dass mindestens ein nichtionischer Öl-in-Wasser-Emulgator in einer Gesamtmenge von 0,2 - 10 Gew.-%, bevorzugt 0,6 - 5 Gew.-%, besonders bevorzugt 1 - 4 Gew.-%, außerordentlich bevorzugt 2 - 3 Gew.-%, jeweils bezogen auf die Gesamtzusammensetzung, enthalten ist.

Ein weiterer Gegenstand der vorliegenden Anmeldung ist die Verwendung der erfindungsgemäßen Öl-in-Wasser-Emulsionen zur Körperpflege und/oder zur nicht-therapeutischen Faltenbehandlung und/oder zur nicht-therapeutischen Behandlung unreiner Haut.

Ein weiterer Gegenstand der vorliegenden Anmeldung ist die Verwendung der erfindungsgemäßen Öl-in-Wasser-Emulsionen zur Herstellung eines Medikaments zur Behandlung der Akne.

Ein weiterer Gegenstand der Erfindung ist die Verwendung von Estern des Pentaerythrits, des Dipentaeryttrits und/oder Tripentaerythrits zur Stabilisierung von Öl-in-Wasser-Emulsionen, die mindestens ein Retinoid enthalten.

### Weitere fakultative Hilfs- und Zusatzstoffe

Je nach Applikationszweck enthalten die kosmetischen Formulierungen eine Reihe weiterer Hilfs-und Zusatzstoffe wie beispielsweise ionische Emulgatoren/Tenside, weitere Wachs- oder Lipidkomponenten, weitere Verdickungsmittel, Überfettungsmittel, Stabilisatoren, Polymere, Lecithine, Phospholipide, biogene Wirkstoffe, UV-Lichtschutzfaktoren, Antioxidantien, Deodorantien, Filmbildner, Quellmittel, Insektenre-pellentien, Hydrotrope, Solubilisatoren, Konservierungsmittel, Parfümöle, Farbstoffe etc., die nachstehend exemplarisch aufgelistet sind. Die Mengen der jeweiligen Zusätze richten sich nach der beabsichtigten Verwendung.

Als weitere Wachs- oder Lipidkomponenten können Fette und fettähnliche Substanzen mit wachsartiger Konsistenz eingesetzt werden. Die erfindungsgemäß als Fette und fettähnliche Substanzen mit wachsartiger Konsistenz bezeichneten Komponenten weisen inen Schmelzpunkt (unter Normalbedingungen = 1013 mbar) von mindestens 30 °C auf. Hierzu gehören u.a. Triglyceridfette, Mono- und Diglyceride von gesättigten, linearen C12-C40-Fettsäuren, natürliche und synthetische Wachse, Fett- und Wachsalkohole, C10-C40-Fettsäuren, Ester von Fettalkoholen und Fettsäuren, die nicht bei 25°C flüssig sind, sowie Fettsäureamide und beliebige Gemische dieser Substanzen. Unter Fetten versteht man Triacylglycerine, also die Dreifachester von Fettsäuren mit Glycerin.

Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln.

Erfindungsgemäß einsetzbar und als Konsistenzgeber besonders gut geeignet sind sogenannte gehärtete Fette und Öle, die durch Partialhydrierung gewonnen werden. Pflanzliche gehärtete Fette und Öle sind bevorzugt, z. B. gehärtetes Rizinusöl, Erdnussöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten Triglycerid-Gemische.

Als zusätzliche Wachskomponenten sind insbesondere Mono- und Diglyceride bzw. Mischungen dieser Partialglyceride einsetzbar. Zu den erfindungsgemäß einsetzbaren Glyceridgemischen zählen die von Cognis vermarkteten Produkte Novata AB und Novata B (Gemisch aus C12-C18-Mono-, Di- und Triglyceriden) sowie Cutina MD oder Cutina GMS (Glycerylstearat).

Mischester sowie Mischungen aus Mono-, Di- und Triglyceriden sind erfindungsgemäß bevorzugt geeignet, da sie eine geringere Neigung zur Kristallisation zeigen und somit die Performance der erfindungsgemäßen Zusammensetzung weiter verbessern.

Erfindungsgemäß besonders bevorzugte Öl-in-Wasser-Emulsionen enthalten daher 2 - 10 Gew.-%, bevorzugt 4 - 8 Gew.-%, besonders bevorzugt 5 - 6 Gew.-% mindestens einer Lipidkomponente, ausgewählt aus Glycerylmonostearat, Glyceryldistearat und Mischungen hiervon.

Zu den erfindungsgemäß einsetzbaren Fettalkoholen zählen die linearen C12-C50-Fettalkohole, insbesondere die C12-C24-Fettalkohole, die aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte, unverzweigte Fettalkohole. Erfindungsgemäß einsetzbar sind auch Fettalkoholschnitte, wie sie bei der Reduktion natürlich vorkommender Fette und Öle wie z. B. Rindertalg, Erdnussöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden.

Erfindungsgemäß bevorzugt geeignet sind C14-C22-Fettalkohole, die beispielsweise von Cognis unter der Bezeichnung Lanette 16 (C16-Alkohol), Lanette 14 (C14-Alkohol), Lanette O (C16/C18-Alkohol) und Lanette 22 (C18/C22-Alkohol) vermarktet werden.

Fettalkohole verleihen den Zusammensetzungen ein trockeneres Hautgefühl als Triglyceride und sind daher gegenüber letzteren bevorzugt.

Erfindungsgemäß besonders bevorzugte Öl-in-Wasser-Emulsionen enthalten daher 1,5 - 6 Gew.-%, bevorzugt 2 - 5 Gew.-%, besonders bevorzugt 3 - 4 Gew.-% mindestens einer Lipidkomponente, ausgewählt aus Myristylalkohol, Cetylalkohol, Stearylalkohol, Arachidylalkohol, Behenylalkohol und Mischungen hiervon.

Als zusätzliche Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-,Lignocerin-, Cerotin-, Melissin-, Eruca-und Elaeostearinsäure sowie substituierte Fettsäuren, wie z. B. 1 2-Hydroxystearinsäure, und die Amide oder Monoethanolamide der Fettsäuren, wobei diese Aufzählung beispielhaften und keinen beschränkenden Charakter hat.

Erfindungsgemäß verwendbar sind außerdem natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die zusätzliche Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, aus der Gruppe der mit Carbonsäuren veresterten Polyole (andere als Pentaerythrit, Dipentaerythrit oder Tripentaerythrit), insbesondere 1,2-Propylenglycol, Ethylenglycol und Glycerin, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Cetylpalmitat, Stearylstearat, Palmitylstearat, Stearylbehenat, Cetearylbehenat und Behenylbehenat einsetzbar. Aus dieser Gruppe der sogenannten Wachsester ist Cetylpalmitat besonders bevorzugt, insbesondere in Mengen von 0,2 - 1 Gew.-%, bevorzugt 0,4 - 0,8 Gew.-%, besonders bevorzugt 0,5 - 0,6 Gew.-%, jeweils bezogen auf das Gewicht der erfindungsgemäßen Öl-in-Wasser-Emulsion.

Als oberflächenaktive Stoffe können anionische, kationische und/oder amphotere bzw. zwitterionische Tenside bzw. Emulgatoren oder ein beliebiges Gemisch dieser Tenside/Emulgatoren enthalten sein. Der Gehalt oberflächenaktiver Substanzen hängt von der Art der Formulierung ab, übersteigt aber üblicherweise 10 Gew.-% nicht. Eine bevorzugte Ausführungsform der Erfindung enthält keine anionischen oder kationischen Tenside, weist aber einen Gehalt an nichtionischen Tensiden auf.

Die nichtionischen Tenside können u.a. auch in den handelsüblichen Gelbildnern enthalten sein. Typische Beispiele für anionische Tenside sind Seifen, Alkylbenzolsulfonate, Alkansulfonate, Olefinsulfonate, Alkylethersulfonate, Glycerinethersulfonate, alpha-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate und Alkyl(ether)phosphate. Sofern die anionischen Tenside Polyglycoletherketten enthalten, können diese eine konventionelle, vorzugsweise- jedoch eine eingeengte Homologenverteilung aufweisen.

Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, alpha-Olefinsulfonate, Ethercarbonsäuren, Alkyloligoglucoside, Fettsäureglucamide, Alkylamidobetaine und/oder Amphoacetate.

Als weitere Verdickungsmittel eignen sich beispielsweise Aerosil-Typen (hydrophile Kieselsäuren), Polysaccharide, insbesondere Xantban-Gum, Guar-Guar, Agar-Agar, Alginate, Carboxymethylcellulose, Hydroxyethylcellulose und Hydroxypropylcellulose, Polyvinylalkohol, Polyvinylpyrrolidon und Bentonite, wie z. B. Bentone Gel VS-5PC (Rheox).

Weitere optionale Wirkstoffe sind beispielsweise Tocopherol, Tocopherolacetat, Tocopherolpalmitat, Ascorbinsäure, beta-Glucane, Retinol, Bisabolol, Allantoin, Phytantriol, Panthenol, alpha-Hydroxycarbonsäuren (AHA-Säuren), Ceramide, Pseudoceramide, essentielle Öle, Pflanzenextrakte und Vitaminkomplexe.

Zur Verbesserung des Fließverhaltens können ferner Hydrotrope, wie beispielsweise Ethanol, Isopropylalkohol oder Polyole eingesetzt werden. Polyole, die hier in Betracht kommen, besitzen vorzugsweise 2 bis 15 Kohlenstoffatome und mindestens zwei Hydroxylgruppen. Bevorzugt sind diese Polyole ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit Xylitol, sowie Mischungen der vorgenannten Substanzen. Als Polyole geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.

Als Konservierungsmittel eignen sich beispielsweise Phenoxyethanol, Parabene, Pentandiol oder Sorbinsäure und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten, Stängeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage. Als Farbstoffe können die für kosmetische Zwecke geeigneten und zugelassenen Substanzen verwendet werden. Beispiele sind Cochenillerot A (C.l. 16255), Patentblau V (C.1.42051), Indigotin (C.1.73015), Chlorophyllin (C.1.75810), Chinolingelb (C.1.47005), Titandioxid (C.1. 77891), Indanthrenblau RS (C.1. 69800) und Krapplack (C.1.58000). Diese Farbstoffe werden üblicherweise in Konzentrationen von 0,001 bis 0,1 Gew.-%, bezogen auf die gesamte Mischung, eingesetzt.

Das folgende Beispiel soll den Gegenstand der vorliegenden Anmeldung erläutern, ohne ihn hierauf zu beschränken.

Tagescreme in Form einer Öl-in-Wasser-Emulsion zur Antifalten-Behandlung und/oder zur Behandlung unreiner Haut und/oder zur Behandlung von Aknehaut

| | |
|---|---|
| PENTAERYTHRITYL DISTEARAT | 1,200000 |
| COCO-CAPRYLATE/CAPRATE | 4,000000 |
| DICAPRYLYL CARBONATE | 3,000000 |
| PROPYLENE GLYCOL | 5,000000 |
| SODIUM POLYACRYLATE | 0,500000 |
| PARFUM | 0,350000 |
| RETINYL PALMITATE | 0,050000 |
| 1,10-DECANEDIOL | 0,020000 |
| 10-HYDROXYDECANOIC ACID | 0,020000 |
| SEBACIC ACID | 0,020000 |
| 1,3-BUTYLENE GLYCOL | 5,000000 |
| TETRASODIUM EDTA | 0,070000 |
| BHT | 0,050000 |
| PHENOXYETHANOL | 1,000000 |
| METHYLPARABEN | 0,400000 |
| PROPYLPARABEN | 0,400000 |
| Wasser | ad 100,000000 |

Die angegebenen Mengen beziehen sich auf Gew.-% des angegebenen Inhaltsstoffes in der Gesamtzusammensetzung.

## Patentansprüche

1. Öl-in-Wasser-Emulsion, enthaltend
(a) mindestens ein Retinoid,
(b) 0,1 - 10 Gew.-% wenigstens einer Wachskomponente mit einem Schmelzpunkt von wenigstens 30°C, ausgewählt aus der Gruppe der Pentaerythritester, der Dipentaerythritester und/oder der Tripentaerythritester sowie Mischungen hiervon,
(c) 1 - 30 Gew.-% wenigstens einer bei 25°C flüssigen Ölkomponente und
(d) 40 - 95 Gew.-% Wasser.

2. Öl-in-Wasser-Emulsion gemäß Anspruch 1, **dadurch gekennzeichnet, dass** die Wachskomponente (b) in einer Menge von 0,2 - 5 Gew.-% der Gesamtzusammensetzung enthalten ist.

3. Öl-in-Wasser-Emulsion gemäß Anspruch 1 oder 2, **dadurch gekennzeichnet, dass** die Wachskomponente (b) ausgewählt ist aus der Gruppe der Ester gesättigter oder ungesättigter und/ oder verzweigter oder unverzweigter C6-C24-Fettsäuren, vorzugsweise C14-C22-Fettsäuren des Pentaerythrits, des Dipentaerythrits und/oder des Tripentaerythrits oder einem beliebigen Gemisch dieser Ester, wobei weniger als 0,3 Gew.-% C17-Fettsäureester enthalten sind.

4. Öl-in-Wasser-Emulsion gemäß einem der Ansprüche 1 bis 3, **dadurch gekennzeichnet, dass** die Wachskomponente (b) ausgewählt ist aus der Gruppe der Ester, die durch Umsetzung des Pentaerythrits oder des Dipentaerythrits mit einem Fettsäuregemisch, enthaltend 40 - 50 Gew.-% C16-Fettsäure und 45 - 55 Gew.-% C18-Fettsäure, erhältlich ist.

5. Öl-in-Wasser-Emulsion gemäß einem der Ansprüche 1 bis 4, **dadurch gekennzeichnet, dass** die Wachskomponente (b) ausgewählt ist aus der Gruppe der Ester des Pentaerythrits mit einem Anteil an (i) 5 - 35 Gew.-% Monoester, (ii) 20 - 50 Gew.-% Diester und (iii) 25 - 50 Gew.-% Triester, und gegebenenfalls Tetraester.

6. Öl-in-Wasser-Emulsion gemäß einem der Ansprüche 1 bis 5, **dadurch gekennzeichnet, dass** die Wachskomponente (b) ausgewählt ist aus der Gruppe der Ester des Pentaerythrits mit einem Anteil an (i) 12 - 19 Gew.-% Monoester, (ii) 25 - 35 Gew.% Diester, (iii) 30 - 40 Gew.-% Triester und (iv) 6 - 11 Gew.-% Tetraester.

7. Öl-in-Wasser-Emulsion gemäß einem der Ansprüche 1 bis 6, **dadurch gekennzeichnet, dass** sie 0,05 - 5 Gew.-% wenigstens eines polymeren Gelbildners, ausgewählt aus der Gruppe der Homopolymere oder Copolymere von Acrylsäure und/oder Acrylamid und/oder Acryloyldimethyltaurat und deren Salzen, enthält.

8. Öl-in-Wasser-Emulsion gemäß einem der Ansprüche 1 bis 7, **dadurch gekennzeichnet, dass** sie
(a) mindestens ein Retinoid,
(b) 0,1 - 10 Gew.-% wenigstens eines C16-C18-Esters des Pentaerythrits und/oder des Dipentaerythrits,
(c) 1 - 30 Gew.-% wenigstens einer bei 25 °C flüssigen Ölkomponente
(d) 0,05 - 5 Gew-% wenigstens eines Polyacrylats und
(e) 40 - 95 Gew.-% Wasser
enthält.

9. Öl-in-Wasser-Emulsion gemäß einem der Ansprüche 1 bis 8, **dadurch gekennzeichnet, dass** sie
(a) mindestens ein Retinoid,
(b) 0,1 - 10 Gew.-% wenigstens eines C16-C18-Esters des Pentaerythrits und/oder des Dipentaerythrits,
(c) 1 - 30 Gew.-% wenigstens einer bei 25 °C flüssigen Ölkomponente,
(d) 0,05 - 5 Gew-% wenigstens eines Acryloyldimethyltaurats und
(e) 40 - 95 Gew.-% Wasser
enthält.

10. Öl-in-Wasser-Emulsion gemäß einem der Ansprüche 1 bis 9, **dadurch gekennzeichnet, dass** die Ölkomponente mindestens ein polares Öl und mindestens ein unpolares Öl enthält, wobei das Gewichtsverhältnis der Gesamtheit der polaren Öle zur Gesamtheit der unpolaren Öle 5:1 bis 20:1, bevorzugt 6:1 - 10:1 und besonders bevorzugt 7:1 - 8:1 beträgt.

11. Öl-in-Wasser-Emulsion gemäß einem der Ansprüche 1 bis 10, **dadurch gekennzeichnet, dass** das mindestens eine Retinoid ausgewählt ist aus Retinol (Vitamin A₁), den C₂-C₂₂-Fettsäureestern des Retinols (= Retinylester, insbesondere Retinylpalmitat und Retinylacetat), 3,4-Didehydroretinol (Vitamin A₂), Retinal und seinen Isomeren, *all-trans-*Retinsäure, 9-*cis*-Retinsäure, 13-*cis*-Retinsäure (Tretinoin), den Estern der Retinsäure sowie synthetischen Retinoiden, insbesondere Isotretinoin, Acitretin, Arotinoiden, Tazarotenen sowie weiterhin Substanzen, die eine spezifische biologische Wirkung durch die Bindung an die Retinoid-Rezeptoren RAR (retinoid acid receptor) und RXR (retinoid X receptor) aufweisen und deren Aktivierung verursachen.

12. Öl-in-Wasser-Emulsion gemäß einem der Ansprüche 1 bis 11, **dadurch gekennzeichnet, dass** keine anionischen und keine kationischen Tenside/Emulgatoren enthalten sind.

13. Verwendung einer Öl-in-Wasser-Emulsion gemäß einem der Ansprüche 1 bis 12 zur Körperpflege und/oder zur nicht-therapeutischen Faltenbehandlung und/oder zur nicht-therapeutischen Behandlung unreiner Haut.

14. Verwendung einer Öl-in-Wasser-Emulsion gemäß einem der Ansprüche 1 bis 12 zur Herstellung eines Medikaments zur Behandlung der Akne.

15. Verwendung von Estern des Pentaerythrits, des Dipentaerythrits und/oder Tripentaerythrits zur Verbesserung der Stabilität von Öl-in-Wasser-Emulsionen, enthaltend mindestens ein Retinoid.

## Claims

1. An oil-in-water emulsion containing
(a) at least one retinoid,
(b) 0,1-10 wt.% at least one wax component with a melting point of at least 30°C selected from the group of pentaerythritol esters, dipentaerythritol esters and/or tripentaerythritol esters and mixtures thereof,
(c) 1-30 wt.% of at least one oil component liquid at 25°C and
(d) 40-95 wt.% of water.

2. An oil-in-water emulsion according to claim 1, **characterised in that** the wax component (b) is present in a quantity of 0.2-5 wt.% of the total composition.

3. An oil-in-water emulsion according to claim 1 or claim 2, **characterised in that** the wax component (b) is selected from the group of esters of saturated or unsaturated and/or branched or unbranched C₆-C₂₄ fatty acids, preferably C₁₄-C₂₂ fatty acids of pentaerythritol, dipentaerythritol and/or tripentaerythritol or any desired mixture of these esters, wherein less than 0.3 wt.% of C₁₇ fatty acid esters are present.

4. An oil-in-water emulsion according to any one of claims 1 to 3, **characterised in that** the wax component (b) is selected from the group of esters obtainable by the reaction of pentaerythritol or dipentaerythritol with a fatty acid mixture containing 40-50 wt.% of C₁₆ fatty acid and 45-55 wt.% of C₁₈ fatty acid.

5. An oil-in-water emulsion according to any one of claims 1 to 4, **characterised in that** the wax component (b) is selected from the group of esters of pentaerythritol with a proportion of (i) 5-35 wt.% of monoesters, (ii) 20-50 wt.% of diesters and (iii) 25-50 wt.% of triesters, and optionally tetraesters.

6. An oil-in-water emulsion according to any one of claims 1 to 5, **characterised in that** the wax component (b) is selected from the group of esters of pentaerythritol with a proportion of (i) 12-19 wt.% of monoesters, (ii) 25-35 wt.% of diesters and (iii) 30-40 wt.% of triesters, and (iv) 6-11 wt.% of tetraesters.

7. An oil-in-water emulsion according to any one of claims 1 to 6, **characterised in that** it contain 0.05-5 wt.% of at least one polymeric gel former selected from the group of homopolymers or copolymers of acrylic acid and/or acrylamide and/or acryloyldimethyltaurate and the salts thereof.

8. An oil-in-water emulsion according to any one of claims 1 to 7, **characterised in that** it contains
(a) at least one retinoid,
(b) 0.1-10 wt.% of at least one C₁₆-C₁₈ ester of pentaerythritol and/or dipentaerythritol,
(c) 1-30 wt.% of at least one oil component liquid at 25°C,
(d) 0.05-5 wt.% of at least one polyacrylate and
(e) 40-95 wt.% of water.

9. An oil-in-water emulsion according to any one of claims 1 to 8, **characterised in that** it contains
(a) at least one retinoid,
(b) 0.1-10 wt.% of at least one C₁₆-C₁₈ ester of pentaerythritol and/or dipentaerythritol,
(c) 1-30 wt.% of at least one oil component liquid at 25°C,
(d) 0.05-5 wt.% of at least one acryloyldimethyltaurate and
(e) 40-95 wt.% of water.

10. An oil-in-water emulsion according to any one of claims 1 to 9, **characterised in that** the oil component contains at least one polar oil and at least one nonpolar oil, wherein the weight ratio of the entirety of the polar oils to the entirety of the nonpolar oils amounts to 5:1-20:1, preferably to 6:1-10:1 and particularly preferably to 7:1-8:1.

11. An oil-in-water emulsion according to any one of claims 1 to 10, **characterised in that** at least one retinoid is selected from retinol (vitamin A₁), the C₂-C₂₂ fatty acid esters of retinol (= retinyl esters, in particular retinyl palmitate and retinyl acetate), 3,4-didehydroretinol (vitamin A₂), retinal and the isomers thereof, *all-trans*-retinoic acid, 9-*cis*-retinoic acid, 13-*cis*-retinoic acid (tretinoin), the esters of retinoic acid and synthetic retinoids, in particular isotretinoin, acitretin, arotinoids, tazarotenes and furthermore substances which exhibit a specific biological action by binding to the retinoid receptors RAR (retinoid acid receptor) and RXR (retinoid X receptor) and bring about the activation thereof.

12. An oil-in-water emulsion according to any one of claims 1 to 11, **characterised in that** no anionic and no cationic surfactants/emulsifiers are present.

13. Use of an oil-in-water emulsion according to any one of claims 1 to 12 for personal hygiene and/or for non-therapeutic wrinkle treatment and/or for non-therapeutic treatment of blemished skin.

14. Use of an oil-in-water emulsion according to any one of claims 1 to 12 for producing a medicament for treating acne.

15. Use of esters of pentaerythritol, dipentaerythritol and/or tripentaerythritol for improving the stability of oil-in-water emulsions containing at least one retinoid.

## Revendications

1. Émulsion huile-dans-eau, contenant :
(a) au moins un rétinoïde ;
(b) à concurrence de 0,1 à 10 % en poids, au moins un composant de cire possédant un point de fusion d'au moins 30°C, choisi parmi le groupe des esters de pentaérythritol, des esters de dipentaérythritol et/ou des esters de tripentaérythritol et de leurs mélanges ;
(c) à concurrence de 1 à 30 % en poids, au moins un composant d'huile liquide à 25°C ; et
(d) à concurrence de 40 à 95 % en poids, de l'eau.

2. Émulsion huile-dans-eau selon la revendication 1, **caractérisée en ce que** le composant de cire (b) est contenu en une quantité de 0,2 à 5 % en poids de la composition totale.

3. Émulsion huile-dans-eau selon la revendication 1 ou 2, **caractérisée en ce que** le composant de cire (b) est choisi parmi le groupe des esters d'acides gras en C₆-C₂₄ saturés ou insaturés et/ou ramifiés ou non ramifiés, de préférence des acides gras en C₁₄-C₂₂ du pentaérythritol, du dipentaérythritol et/ou du tripentaérythritol ou de n'importe quel mélange de ces esters, des esters d'acides gras en C₁₇ étant contenus à concurrence de moins de 0,3 % en poids.

4. Émulsion huile-dans-eau selon l'une quelconque des revendications 1 à 3, **caractérisée en ce que** le composant de cire (b) est choisi parmi le groupe des esters que l'on obtient par mise en réaction du pentaérythritol ou du dipentaérythritol avec un mélange d'acides gras contenant un acide gras en C₁₆ à concurrence de 40 à 50 % en poids et un acide gras en C₁₈ à concurrence de 45 à 55 % en poids.

5. Émulsion huile-dans-eau selon l'une quelconque des revendications 1 à 4, **caractérisée en ce que** le composant de cire (b) est choisi parmi le groupe des esters du pentaérythritol avec une fraction (i) de monoesters à concurrence de 5 à 35 % en poids, (ii) de diesters à concurrence de 20 à 50 % en poids et (iii) de triesters à concurrence de 25 à 50 % en poids, et le cas échéant de tétraesters.

6. Émulsion huile-dans-eau selon l'une quelconque des revendications 1 à 5, **caractérisée en ce que** le composant de cire (b) est choisi parmi le groupe des esters du pentaérythritol avec une fraction (i) de monoesters à concurrence de 12 à 19 % en poids, (ii) de diesters à concurrence de 25 à 35 % en poids, (iii) de triesters à concurrence de 30 à 40 % en poids, et (iv) de tétraesters à concurrence de 6 à 11 % en poids.

7. Émulsion huile-dans-eau selon l'une quelconque des revendications 1 à 6, **caractérisée en ce qu'**elle contient, à concurrence de 0,05 à 5 % en poids, au moins un gélifiant polymère choisi parmi le groupe des homopolymères ou des copolymères d'acide acrylique et/ou d'acrylamide et/ou d'acryloyldiméthyltaurate et de leurs sels.

8. Émulsion huile-dans-eau, selon l'une quelconque des revendications 1 à 7, **caractérisée en ce qu'**elle contient :
(a) au moins un rétinoïde ;
(b) à concurrence de 0,1 à 10 % en poids, au moins un ester en C₁₆-C₁₈ du pentaérythritol et/ou du dipentaérythritol ;
(c) à concurrence de 1 à 30 % en poids, au moins un composant d'huile liquide à 25°C ;
(d) à concurrence de 0,05 à 5 % en poids, au moins un polyacrylate ; et
(e) à concurrence de 40 à 95 % en poids, de l'eau.

9. Émulsion huile-dans-eau, selon l'une quelconque des revendications 1 à 8, **caractérisée en ce qu'**elle contient :
(a) au moins un rétinoïde ;
(b) à concurrence de 0,1 à 10 % en poids, au moins un ester en C₁₆-C₁₈ du pentaérythritol et/ou du dipentaérythritol ;
(c) à concurrence de 1 à 30 % en poids, au moins un composant d'huile liquide à 25°C ;
(d) à concurrence de 0,05 à 5 % en poids, au moins un acryloyldiméthyltaurate ; et
(e) à concurrence de 40 à 95 % en poids, de l'eau.

10. Émulsion huile-dans-eau, selon l'une quelconque des revendications 1 à 9, **caractérisée en ce que** le composant d'huile contient au moins une huile polaire et au moins une huile apolaire, la proportion de la totalité des huiles polaires par rapport à la totalité des huiles apolaires s'élevant de 5:1 à 20:1, de préférence de 6:1 à 10:1 et de manière particulièrement préférée de 7:1 à 8:1.

11. Émulsion huile-dans-eau, selon l'une quelconque des revendications 1 à 10, **caractérisée en ce qu'**elle contient au moins un rétinoïde choisi parmi le rétinol (vitamine A₁), les esters d'acides gras en C₂-C₂₂ du rétinol (= ester rétinylique, en particulier le palmitate de rétinyle et l'acétate de rétinyle), le 3,4-didéshydrorétinol (vitamine A₂), le rétinal et ses isomères, l'acide rétinoïque *tout trans,* l'acide 9-*cis*-rétinoïque, l'acide 13-*cis-*rétinoïque (trétinoïne), les esters de l'acide rétinoïque ainsi que des rétinoïdes synthétiques en particulier l'isotrétinoïne, l'acitrétine, les arotinoïdes, les tazarotènes ainsi que d'autres substances qui présentent un effet biologique spécifique via la liaison aux récepteurs du rétinoïde RAR (récepteur de l'acide rétinoïque) et RXR (récepteur du rétinoïde X) et qui déclenchent l'activation desdits récepteurs.

12. Émulsion huile-dans-eau, selon l'une quelconque des revendications 1 à 11, **caractérisée en ce qu'**elle ne contient aucun agent tensioactif/émulsifiant anionique et aucun agent tensioactif/émulsifiant cationique.

13. Utilisation d'une émulsion huile-dans-eau, selon l'une quelconque des revendications 1 à 12, pour les soins corporels et/ou pour le traitement non thérapeutique des rides et/ou pour le traitement non thérapeutique d'une peau qui n'est pas pure.

14. Utilisation d'une émulsion huile-dans-eau, selon l'une quelconque des revendications 1 à 12, pour la préparation d'un médicament destiné au traitement de l'acné.

15. Utilisation d'esters du pentaérythritol, du dipentaérythritol et/ou du tripentaérythritol pour améliorer la stabilité d'émulsions huile-dans-eau contenant au moins un rétinoïde.
